# EUROPEAN PATENT APPLICATION

(11) **EP 3 741 382 A1**
(43) Date of publication of application: **25.11.2020**
(21) Application number: 18781670.7
(22) Date of filing: 01.03.2018
(51) Int. Cl.: A61K 38/08, C07K 7/06, A61P 1/00, A61P 3/10, A61P 25/00, A61P 31/00, A61P 35/00

(54) **SPECIFIC PEPTIDE CLATHRIN INHIBITORS**

(30) Priority: 07.04.2017 RU 2017111896
(71) Applicant: Kotin, Oleg Arkadyevich, Gatchina 188300 (RU)
(72) Inventor: KOTIN, Arkadiy Mihaylovich, St.Petersburg 196128 (RU); EMELYANOV, Maksim Olegovich, Samara Region PGT Volzhskiy 446394 (RU); KOTIN, Oleg Arkadyevich, Samara Region PGT Volzhskiy 446394 (RU)
(74) Representative: Schulz Junghans Patentanwälte PartGmbB
(86) International application number: PCT/RU2018/000121
(87) International publication number: WO 2018/186770

(57) **Abstract**

The invention relates to the chemical and pharmaceutical industry. The use of synthetic peptides as specific clathrin inhibitors has been proposed. Also proposed is a method for the prevention or treatment of a disease or condition in an animal, comprising administering an effective amount of peptides or a prodrug or a physiologically acceptable salt or solvate of the above peptides, or a pharmaceutical composition containing the above peptides as specific clathrin inhibitors. Peptides can be used in medicine, veterinary medicine and pharmacology to create new drugs that are specific clathrin inhibitors.

## Description

The present invention relates to biochemistry and more particularly, to biologically active peptides which are specific inhibitors of clathrin and may find application in medicine and pharmacology as drugs for the prevention or treatment of various diseases and physiological conditions responsive to inhibition of clathrin or for which inhibition of clathrin necessary.

The term "clathrin" as used herein, means the intracellular protein, which is the main component of the shell bordered bubbles formed during receptor-mediated endocytosis [McMahon, Boucrot, 2011]. In a classic embodiment, it consists of three clathrin heavy chain (-190 kDa), each of which is closely linked to the light chain (∼ 25 kDa). Heavy chains are encoded by two genes: on chromosome 17 (heavy chain C 17) and on chromosome 22 (heavy chain C22). The latter is represented mainly in the muscles, while C17 is presented in clathrin all cells. However, the two heavy chains have a high degree of homology (95% amino acid sequence overlap) and functionally equivalent [Hood, Royle, 2009]. The polymerization clathrin forms a closed three-dimensional network resembling a sphere with the size of clathrin-coated vesicles, approximately 100 nm. After the formation of vesicles and separation by membrane vesicles (under the influence GTPase dynamin) clathrin-coated shell rapidly dissociates clathrin and can be reused for endocytosis and exocytosis. The process is stimulated by interaction with the specific receptor ligand [McMahon, Boucrot, 2011]. It is crucial that the shutdown of the heavy chain of clathrin activity entails the loss of activity of a clathrin-coated complex [Haar ter, Harrison, Kirchhausen, 2000]. As a result, in clathrin-coated endocytic cell receptors fall not only, but also other metabolites: hormones, neurotransmitters, various proteins [Most et al., 2003]. Also as a result of endocytosis in clathrin-coated cell fall different pathogenic agents such as viruses, toxins and symbiotic microorganisms. For example, the botulinum neurotoxins and tetanus neurotoxin are bacterial proteins that cause two serious diseases: tetanus and botulism. Their action depends on internalization through endocytosis neurotoxin into the nerve endings [Pellett et al., 2015].

There are various drugs, which are inhibitors of clathrin. All of them have one or other defects which restricts the possibilities of their use in medicine.

In particular, the known synthetic preparations having at its core a compound which can bind to terminal domain clathrin with at least one or more amino acids: Ile 52 Ile 62, Ile 80, Phe 91 and / or Ile 93, (WO2013010218A9; Robertson et al., 2014). A significant disadvantage of these compounds is the lack of specificity [Willox, Sahraoui, Royle, 2014].

Also known as an inhibitor of clathrin peptide Thr-Pro-Val-Leu-Glu-Thr-Pro-Lys-Leu-Leu-Leu-Trp (JP2009250552A). However, in this case proposed 12-membered inhibitor sequence is not direct, as originally inhibits binding protein clathrin GRP78, which is characterized by features that are not associated with clathrin. Therefore, the disadvantage of this compound is also a lack of specificity. Furthermore, the proposed sequence consists solely of natural L-amino acids and, therefore, susceptible to endopeptidases, which limits the possibility of its use in vivo.

In order to eliminate these shortcomings by experimentation it was determined that synthetic peptides with non-narcotic type analgesic action, protected earlier by Patents RU2508295, US 9260482 (PCT WO/2013/141750, 2013), effective for different methods of administration (the suboccipital, intramuscular, intranasal and iv) in different models of acute and tonic pain, are specific inhibitors of clathrin.

We have previously demonstrated that the peptide sequence designed not to affect calcium metabolism by binding with tetracycline test do not have immune effects, do not form fibrils in solution, stable in storage and are cheap to manufacture and use (Patent RU2508295).

The problem to be solved by the proposed invention is to extend the range of effective means, are specific inhibitors of clathrin having no side effects and obtained by simple synthesis. The problem is solved by the fact that

To achieve this result, it is proposed the use of a synthetic peptide of general formula (I) [SEQ ID NO: 1]

XDL - XDL1 - XDL2 - L-Lys - L-Leu - XDL3 - L-Thr - R2 (I),

wherein:
XDL - the absence of an amino acid or L-Tyr,
XDL1 - one of the amino acids: L-Leu, L-Ala or D-Ala,
XDL2 - one of the amino acids: L-His, D-His, L-Ala or D-Ala,
XDL3 - one of the amino acids: L-Gln, L-Ala or D-Ala;
R2 - OMe or NH₂,
or peptide - retroinversion of formula (I), having a reverse amino acid sequence with the replacement of L-form amino acids to D-form and D-form amino acids to L-form, of the general formula (II) [SEQ ID NO: 2]

D-Thr - XDL4 - D-Leu - D-Lys - XDL5 - XDL6 - XDL7 - R2 (II)

wherein:
XDL4 - one of the amino acids: D-Gln, D-Ala or L-Ala;
XDL5 - one of the amino acids: D-His, L-His, D-Ala or L-Ala,
XDL6 - one of the amino acids: D-Leu, D-Ala or L-Ala,
XDL7 - the absence of an amino acid or D-Tyr,
R2 - OMe or NH₂,
as a specific inhibitor of clathrin.

Also offered the method of preventing or treating a disease or condition in an animal comprising administering an effective amount of synthetic peptides of general formula (I) [SEQ ID NO: 1] or the general formula (II) [SEQ ID NO: 2] or a prodrug thereof, or a physiologically acceptable salt or solvate of the aforementioned peptides or pharmaceutical composition containing the above peptides as specific inhibitors of clathrin.

The list of diseases in which inhibition of clathrin function has a key role is given in Table 1 below.

**Table 1.**

| **Diseases** | **Reference** |
|---|---|
| Abeta-lipoproteinema | [Aridor, Hannan, 2000] |
| Aceruloplasminemia | [Aridor, Hannan, 2002] |
| Acquired-immunodeficiency syndrome (AIDS) (Human immunodeficiency virus (HIV)) | [Patent WO 2011088431 A1] |
| Acute lymphoblastic leukemia or acute myeloid leukemia | [Aridor, Hannan, 2000] |
| Acute myeloid leukemia | [Aridor, Hannan, 2000] |
| Adeno-associated virus | [Patent WO 2011088431 A1] |
| ADPKD-autosomal dominant polycystic kidney disease | [Aridor, Hannan, 2000] |
| African swine fever (African swine fever virus) | [Patent WO 2011088431 A1] |
| Alpha-1-antichymotrypsin (ACTH) deficiency | [Aridor, Hannan, 2000] |
| Alzheimer disease | [Aridor, Hannan, 2000] |
| Amyotrophic lateral sclerosis | [Aridor, Hannan, 2000] |
| Anderson disease | [Aridor, Hannan, 2002] |
| Anthrax (Anthrax toxin from Bacillus anthracis) | [Patent WO 2011088431 A1] |
| Anti-phospholipid syndrome | [Aridor, Hannan, 2000] |
| Argentine hemorrhagic fever (Junin arenavirus (JUNV)) | [Patent WO 2011088431 A1] |
| Aspartylglucosaminuria | [Aridor, Hannan, 2002] |
| Ataxia telangiectasia | [Aridor, Hannan, 2000] |
| Autosomal dominant retinitis pigmentosa | [Aridor, Hannan, 2002] |
| Autosomal recessive juvenile parkinsonism | [Aridor, Hannan, 2002] |
| Autosomal recessive primary hyperoxaluria | [Aridor, Hannan, 2000] |
| Bare lymphocyte syndrome | [Aridor, Hannan, 2000] |
| Batten disease (neuronal ceroid-lipofuscinosis) | [Aridor, Hannan, 2000] |
| Bluetongue disease/ catarrhal fever (Bluetongue virus-1) | [Patent WO 2011088431 A1] |
| Botulism | [Pellett 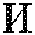 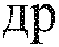 ., 2015] |
| Bovine respiratory disease (eukotoxin (LKT) from Mannheimia haemolytica) | [Patent WO 2011088431 A1] |
| Brugada syndrome | [Aridor, Hannan, 2002] |
| Candidiasis (Candida albicans) | [Patent WO 2011088431 A1] |
| Carcinomas/ haemangio-endotheliomas of the bladder (Bovine papillomavirus BP VI) | [Patent WO 2011088431 A1] |
| Cardiomyopathy | [Aridor, Hannan, 2002] |
| CDG-1a (Congenital Disorder of Glycosylation) | [Aridor, Hannan, 2002] |
| CDG-1b (Congenital Disorder of Glycosylation) | [Aridor, Hannan, 2002] |
| CDG-1c (Congenital Disorder of Glycosylation) | [Aridor, Hannan, 2002] |
| CDG-1d (Congenital Disorder of Glycosylation) | [Aridor, Hannan, 2002] |
| CDG-1e (Congenital Disorder of Glycosylation) | [Aridor, Hannan, 2002] |
| CDG-1f (Congenital Disorder of Glycosylation) | [Aridor, Hannan, 2002] |
| CDG-2a (Congenital Disorder of Glycosylation) | [Aridor, Hannan, 2002] |
| CDG-2b (Congenital Disorder of Glycosylation) | [Aridor, Hannan, 2002] |
| CDG-2c (Congenital Disorder of Glycosylation) (leukocyte adhesion deficiency II syndrome) | [Aridor, Hannan, 2002] |
| CDG-2d (Congenital Disorder of Glycosylation) | [Aridor, Hannan, 2002] |
| Charcot-Marie-Tooth syndrome | [Aridor, Hannan, 2000] |
| Chediak-Higashi syndrome | [Aridor, Hannan, 2000] |
| Chikungunya disease (Chikungunya virus (CHIKV)) | [Patent WO 2011088431 A1] |
| Chlamydia (Chlamydia psittaci and Chlamydia trachomatis) | [Patent WO 2011088431 A1] |
| Choroideremia | [Aridor, Hannan, 2000] |
| Chronic myelomonocytic leukemia | [Aridor, Hannan, 2002] |
| Combined factors V and VIII deficienc | [Aridor, Hannan, 2000] |
| Common cold (Human rhinovirus 2 (HRV)) | [Patent WO 2011088431 A1] |
| Congenital hypothyroidism | [Aridor, Hannan, 2000] |
| Congenital Long QT syndrome | [Aridor, Hannan, 2000] |
| Congenital nephritic syndrome of the finnish type | [Aridor, Hannan, 2002] |
| Congenital sucrase-isomaltase deficiency | [Aridor, Hannan, 2000] |
| Crigler-Najjar | [Aridor, Hannan, 2000] |
| Cushing's disease | [Aridor, Hannan, 2000] |
| Cystic fibrosis | [Aridor, Hannan, 2000] |
| Danon disease | [Aridor, Hannan, 2000] |
| Dengue fever (Dengue virus 1 & 2 (DENV)) | [Patent WO 2011088431 A1] |
| Diabetes mellitus | [Aridor, Hannan, 2000] |
| Diabetes/Wolcott- Rallison syndrome | [Aridor, Hannan, 2002] |
| Diptheria (Corynebacterium diphtheriae) | [Patent WO 2011088431 A1] |
| Dubin-Johnson syndrome | [Aridor, Hannan, 2002] |
| Ebola hemorrhagic fever (Zaire Ebolavirus) | [Patent WO 2011088431 A1] |
| Echovirus disease (Echovirus (EV1) from Picornaviridae) | [Patent WO 2011088431 A1] |
| Ehlers-Danlos syndrome | [Aridor, Hannan, 2002] |
| Epilepsia | [Casillas-Espinosa, Powell, O'Brien, 2012] |
| Equine infectious anemia (Equine infectious anemia virus (EIAV)) | [Patent WO 2011088431 A1] |
| Fabri disease | [Aridor, Hannan, 2000] |
| Faciogenital dysplasia (Aarskog-Scott syndrome) | [Aridor, Hannan, 2002] |
| Familial chylomicronemia | [Aridor, Hannan, 2000] |
| Familial hypercholesterolemia | [Aridor, Hannan, 2000] |
| Familial intrahepatic cholestasis | [Aridor, Hannan, 2000] |
| Feline infectious peritonitis (Feline infectious peritonitis virus (FIPV)) | [Patent WO 2011088431 A1] |
| Frontotemporal dementia with Parkinsonism | [Aridor, Hannan, 2000] |
| Gastroenteritis (Rotavirus) | [Patent WO 2011088431 A1] |
| Genital warts/ cervical cancer (Human papillomavirus 31 (HPV31) and 16 (HPV16)) | [Patent WO 2011088431 A1] |
| Golden staph infections (Staphylococcus aureus) | [Patent WO 2011088431 A1] |
| Griscelli disease | [Aridor, Hannan, 2000] |
| Griscelli disease with hemophagic syndrome | [Aridor, Hannan, 2000] |
| Hepatitis B (Hepatitis B virus (HBV)) | [Patent WO 2011088431 A1] |
| Hepatitis C (Hepatitis C virus (HCV)) | [Patent WO 2011088431 A1] |
| Hereditary emphysema | [Aridor, Hannan, 2000] |
| Hereditary emphysema with liver injury | [Aridor, Hannan, 2000] |
| Hereditary familial amyloidosis of Finish | [Aridor, Hannan, 2002] |
| Hereditary hemochromatosis | [Aridor, Hannan, 2000] |
| Hereditary hyperhomocysteinemia | [Aridor, Hannan, 2002] |
| Hereditary hypofibrinogenemia | [Aridor, Hannan, 2000] |
| Hereditary pancreatitis | [Aridor, Hannan, 2000] |
| Hereditary spherocytosis | [Aridor, Hannan, 2002] |
| Hermansky-Pudlak syndrome type 1 | [Aridor, Hannan, 2000] |
| Hermansky-Pudlak syndrome type 2 | [Aridor, Hannan, 2000] |
| Herpes simplex (Herpes Simplex virus) | [Patent WO 2011088431 A1] |
| Huntington and spinocerebellar ataxias | [Aridor, Hannan, 2002] |
| Huntington's disease | [Aridor, Hannan, 2000] |
| I cell disease | [Aridor, Hannan, 2000] |
| Infantile neuronal ceroid lipofuscinosis | [Aridor, Hannan, 2002] |
| Influenza (Influenza virus) | [Patent WO 2011088431 A1] |
| Jaagsiekte/ ovine pulmonary adenocarcinoma (Jaagsiekte sheep retrovirus (JSRV)) | [Patent WO 2011088431 A1] |
| Kaposi's sarcoma (Kaposi's sarcoma-associated herpes-virus) | [Patent WO 2011088431 A1] |
| Korean hemorrhagic fever (Hantaan virus) | [Patent WO 2011088431 A1] |
| Laron syndrome | [Aridor, Hannan, 2000] |
| Limb-girdle muscular dystrophy | [Aridor, Hannan, 2002] |
| Lissencephaly | [Aridor, Hannan, 2000] |
| Listeriosis (Listeria monocytogenes) | [Patent WO 2011088431 A1] |
| Low plasma lipoprotein a levels | [Aridor, Hannan, 2000] |
| Microvillus inclusion disease | [Aridor, Hannan, 2000] |
| Miller Dieker syndrome | [Aridor, Hannan, 2000] |
| Mohr Tranebjaerg syndrome (human deafness dystonia syndrome) | [Aridor, Hannan, 2000] |
| Motor neuron disease | [Aridor, Hannan, 2000] |
| Mucolipidosis IV | [Aridor, Hannan, 2000] |
| Mucopolysaccharidosis type IV/Maroteaux-Lamy syndrome | [Aridor, Hannan, 2002] |
| Multiple exostoses syndrome | [Aridor, Hannan, 2002] |
| Murine norovirus disease (Murine norovirus-1 (MNV-1)) | [Patent WO 2011088431 A1] |
| Myelodysplastic syndrome | [Aridor, Hannan, 2000] |
| Myeloid leukemia | [Aridor, Hannan, 2000] |
| Neuropathic pain | [Richards, Whittle, Buchbinder, 2012] |
| Nephrogenic diabetes insipidus | [Aridor, Hannan, 2000] |
| Neurohypophyseal diabetes insipidus | [Aridor, Hannan, 2000] |
| Niemann-Pick disease | [Aridor, Hannan, 2000] |
| Oculocerebro-renal syndrome of Lowe | [Aridor, Hannan, 2000] |
| Oculocutaneous albinism | [Aridor, Hannan, 2000] |
| Optiz syndrome | [Aridor, Hannan, 2000] |
| Osteogenesis imperfecta (OI) | [Aridor, Hannan, 2000] |
| Overlap connective tissue disease | [Aridor, Hannan, 2000] |
| Parvo (Canine parvovirus) | [Patent WO 2011088431 A1] |
| Pelizaeus-Merzbacher disease | [Aridor, Hannan, 2000] |
| Pendred syndrome | [Aridor, Hannan, 2002] |
| Periodontitis (Porphyromonas gingivalis) | [Patent WO 2011088431 A1] |
| Persistent hyperinsulinemic hypoglycemia of infancy | [Aridor, Hannan, 2002] |
| Poliomyelitis (Poliovirus) | [Patent WO 2011088431 A1] |
| Polyneuropathy, pigmented retinitis, seronegative polyarthritis, interstitial pulmonary fibrosis, Raynaud's phenomenon, Wegner's granulomatosis, proteinuria | [Aridor, Hannan, 2002] |
| Primary ciliary dyskinesia (Kartagener's syndrome) | [Aridor, Hannan, 2000] |
| Primary hypothyroidism | [Aridor, Hannan, 2000] |
| Protein C deficiency | [Aridor, Hannan, 2000] |
| Pseudoachondroplasia and Multiple epiphyseal | [Aridor, Hannan, 2002] |
| Pseudotuberculosis (Yersinia pseudotuberculosis) | [Patent WO 2011088431 A1] |
| Respiratory infection (Adenovirus) | [Patent WO 2011088431 A1] |
| Rhizomelic chondrodysplasia punctata | [Aridor, Hannan, 2000] |
| Sarcoma | [Aridor, Hannan, 2002] |
| Scott syndrome | [Aridor, Hannan, 2000] |
| Semliki forest virus disease (Semliki forest virus (SFV)) | [Patent WO 2011088431 A1] |
| Simian virus 40 (SV-40) | [Patent WO 2011088431 A1] |
| Sjogren's syndrome | [Aridor, Hannan, 2000] |
| Sphingolipid activator protein deficiency | [Aridor, Hannan, 2000] |
| Sphingolipidoses, gangliosidosis, Sandhoff disease, Fabry disease | [Aridor, Hannan, 2000] |
| Spinal and bulbar muscular atrophy | [Aridor, Hannan, 2000] |
| Spondyloepiphyseal dysplasia congenita | [Aridor, Hannan, 2000] |
| Stargardt-like macular dystrophy | [Aridor, Hannan, 2002] |
| Stif-man syndrome | [Aridor, Hannan, 2000] |
| Shaking Palsy | [Edvardson 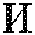 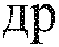 ., 2012] |
| Swine atrophic rhinitis ( Dermonecrotic toxin (DNT) from Bordetella) | [Patent WO 2011088431 A1] |
| Tangier disease | [Aridor, Hannan, 2000] |
| Tay-Sachs | [Aridor, Hannan, 2000] |
| Tetanus | [Pellett 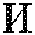 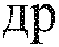 ., 2015] |
| Thyroxine binding globulin deficiency | [Aridor, Hannan, 2000] |
| Torsion dystonia | [Aridor, Hannan, 2002] |
| Tuberous sclerosis | [Aridor, Hannan, 2000] |
| Type I hereditary angioedema | [Aridor, Hannan, 2000] |
| Urinary tract infection (Uropathogenic Escherichia coli) | [Patent WO 2011088431 A1] |
| Usher's syndrome | [Aridor, Hannan, 2000] |
| Vesicular stomatitis (Vesicular stomatitis virus (VSV)) | [Patent WO 2011088431 A1] |
| Viral myocarditis/ CNS infection/ respiratory disease (Coxsackievirus B3 (CVB3) and A9 (CVA9)) | [Patent WO 2011088431 A1] |
| von Willebrand disease type IIA | [Aridor, Hannan, 2000] |
| Wiskott-Aldrich syndrome | [Aridor, Hannan, 2000] |
| X-linked adrenoleukodystrophy | [Aridor, Hannan, 2000] |
| X-linked Charot-Marie-tooth disease | [Aridor, Hannan, 2002] |
| X-linked hypophosphatemia | [Aridor, Hannan, 2002] |
| Zellweger syndrome (cerebro-hepato-renal syndrome) | [Aridor, Hannan, 2000] |

The invention consists in the fact that it has been found experimentally that the claimed peptides having a simple structure (which facilitates their preparation by chemical means), are specific inhibitors of clathrin.

The term "inhibitor of clathrin" as used herein, and variations thereof, means a compound which interacts with clathrin and at least partially inhibit the function of clathrin activity in clathrin-mediated endocytosis (CME), which can be expressed by a decrease CME and/or levels of cells in CME. Thus, inhibition of clathrin in accordance with the invention may be full or partial inhibition of the functional activity of clathrin.

Clathrin-mediated endocytosis (CME) is important not only for the transfer of metabolites in a cell, but also the transmission signals of the cells, and inhibition of the function of clathrin has application in the prevention or treatment of various diseases and conditions. The list of diseases in which inhibition of clathrin function has a key role, published earlier in [Aridor, Hannan, 2002; Aridor, Hannan, 2000], the contents of which are incorporated herein by reference in their entirety.

In particular, inhibitors of clathrin features may find application in many diseases in the central nervous system, including: Alzheimer's disease [Harold et al, 2009], Parkinson's disease [Edvardson et al, 2012], epilepsy [Casillas-Espinosa, Powell, O'Brien, 2012]. An example of a new antiepileptic drug acting on the mechanism of inhibition of processes connected with clathrin is levetiracetam (Keppra ™) [Nowack et al., 2011].

Inhibition of clathrin - mediated endocytosis through clathrin blocking reduces synaptic transmission, reducing the availability of synaptic vesicles that may be used for the prevention or treatment of pain. For example, anticonvulsants such as phenytoin and gabapentin, are very effective in the treatment of neuropathic pain [Richards, Whittle, Buchbinder, 2012]. These drugs act by altering the transmission process of synaptic vesicles, i.e. inhibition by blocking CME clathrin may stop or limit the transmission of pain signals and thereby improve or reduce the sensation of pain.

Also of particular interest is evidence that one of the mutations in the heavy chain of clathrin leads to a rare disease in people - the loss of the pain and tactile sensitivity [Nahorski et al, 2015.], And that the intensity of inflammation and, consequently, pain reactions It is dependent on clathrin heavy chain [Escobar et al., 2006; Kim, Sorg, Arrieumerlou, 2011].

In fact, the prevention or treatment of any physiologic disorder which can be effected by inhibiting CME, as described herein, directly covered by the present invention.

Also very important is the ability to suppress inhibitors of clathrin excessive cell proliferation in cancer. Several currently known inhibitors, interacting with mitotic proteins are currently in preclinical or clinical trials as agents for the treatment of cancer. Antimitotic effects of these inhibitors may be due in part to the indirect blocking clathrin-mitotic functions [Booth et al., 2011]. Consequently, clathrin inhibitors are not only useful molecular tools for solving a clathrin role in the mitotic cycle, but at least in some embodiments, can be used as antimitotic compounds for the treatment of cancer and cell proliferative disorders. In this connection it should be noted that many growth factor receptors (e.g., EGF-R) also require clathrin-mediated endocytosis (CME) for internalization and maintain cell activity. Blocking CME prevents cell proliferation in these cases and is further evidence of cancer and anti-proliferative activity of clathrin inhibitors [Smith et al., 2013].

Also inhibition CME in accordance with one or more embodiments of the invention is important to develop means to prevent penetration of the cells, including the brain, a number of pathogenic viruses as well as all kinds of toxins, including peptide, which penetrate into the cell by endocytosis. Among these viruses, and toxins include (but not be limited to): diphtheria, leukotoxin (LKT), chlamydia, anthrax toxin, Staphylococcus aureus, Candida, periodontitis, adenovirus, African swine fever virus, fever virus, dengue, Zaire ebolavirus (hemorrhagic fever Ebola), hepatitis B virus, hepatitis C virus associated with Kaposi's sarcoma herpes virus, human immunodeficiency virus (HIV), influenza virus, and others. For example, the mechanism of action of the most active compounds to prevent infektsirovaniya Ebola virus, associated with blocking of adhesion of viruses on a cell surface, or the suppression of their endocytosis into the cell [Anantpadma et al, 2016.; Aleksandrowicz et al, 2011.; Piccini, Castilla, Damonte, 2015].

Thus, examples of specific diseases and conditions to which the present invention finds application for the prevention or treatment include, but are not limited to cell proliferative diseases and conditions, multifocal leukoencephalopathy, polycystic kidney disease, a disease associated with amyloid β- (such as Alzheimer's disease), neurodegenerative disorders, neuropsychiatric disorders, psychotic disorders, psychoses, bipolar disorders, schizophrenia, aberrant unregulated th excitation of neurons, seizures, neuropathic pain, migraine, and all diseases and conditions mediated by or otherwise associated with a synaptic signal transduction processes involving CME (such as epilepsy) or with impaired cellular vesicle recycling. Also, as indicated above, inhibition of the CME in accordance with one or more embodiments of the invention may also be useful in preventing the penetration of pathogenic agents in a number of cells and thus be useful in preventing or treating diseases or conditions associated with it.

All peptides claimed family have previously shown analgesic effect (see. PCT WO/2013/141750), and are specific inhibitors of clathrin.
Escription illustrated by the following figures:
Fig. 1 - chromatogram of Peptide (sequence of the six amino acids);
Fig. 2 - eluting the protein fraction;
Fig. 3 - majeure proteinaceous compound;
Fig. 4 - multicolored protein profile to determine the approximate mass during gel electrophoresis (Thermo Fisher Scientific) (taken from the official website of the company «Thermo Fisher Scientific)) / [Electronic resource]. - Access: URL: https://www.thermofisher.com/order/catalog/product/26634, treatment date: 17.03.2017). Synthesis of peptides was performed by methods of peptide chemistry by solid phase method using the L and D amino acids.

### EXAMPLE 1. Synthesis of the peptide L-Leu-D-His-L-Lys-L-Leu-L-Gln-L-Thr-NH₂ (Peptide Nº7 SEQ ID N0:1).

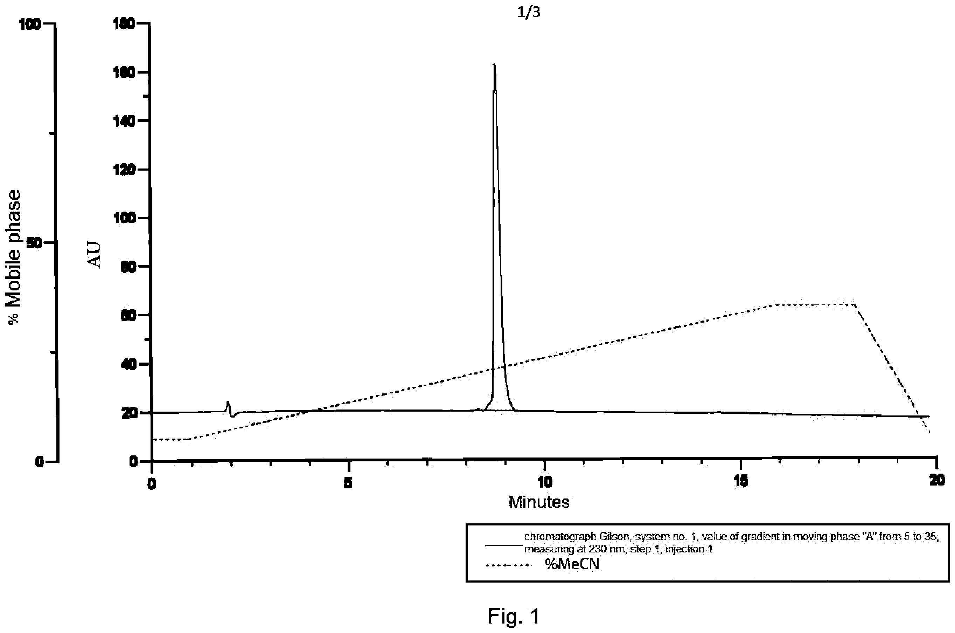

Peptide L-Leu-D-His-L-Lys-L-Leu-L-Gln-L-Thr-NH₂ was prepared by automatic solid-phase synthesis of Fmoc-Rink scheme on the polymer (Rink Amide Resin, 0,6 mmol amino- groups per 1 g of resin) using DCC / HOBt (N, N'-dicyclohexylcarbodiimide / 1-hydroxybenzotriazole) method, the amino acid activation. Deprotection was performed by treatment with piperidine / DMF solution (piperidine / N,N-dimethylformamide) (1:4) for 7 minutes. The protection groups of the side chains produced following groups: tBu (tert-butylether) for tyrosine, threonine, Trt (trityl or triphenylmethyl) for glutamine and histidine, Boc (t-butyloxycarbonyl) lysine. Peptides were cleaved from the resin and deprotected with a mixture of TFA / H2O / EDT (trifluoroacetic acid / water / 1,2-ethanedithiol) (90: 5: 5). Purification of the peptides was done by reverse phase HPLC (C18 column) eluent - acetonitrile - water (with 0.1 M potassium dihydrogen phosphate) in a ratio of 6:4. Peptides were characterized using mass spectrometer. Peptide purity was checked as HPLC, column Waters DeltaPak C18 3,9*150 mm 5µm 100Å; solution A: 0,1% TFA in 100% water / MeCN; flow rate - 1 mL/min; detection wavelength - 230nm (Figure 1).

### EXAMPLE 2. Testing the synthesized peptides as the activity of specific inhibitors of clathrin.

The basis of the experiment based on a procedure of affinity chromatography, where the stationary phase acts as a peptide molecule attached to the special matrix. The mobile phase acts as a rat brain extract. The protein fraction, specifically bound to the peptide molecule, subsequently subjected to fractionation by electrophoresis and direct detection of the individual protein molecules.

Affinity adsorbent was prepared as follows: a matrix for the sorbent was cyanogen bromide-activated sepharose 4B (Sigma-Aldrich), dissolved in 1 mM hydrochloric acid (HCl). To conduct research on the possibility of clathrin binding peptide, namely peptide for landing on a carrier (sepharose) was necessary to introduce a spacer. Task 'spacer' - spatially divided carrier (sepharose) and the actual peptide molecule to create the conditions of interaction of the test peptide and proteins brain extract. This use of "spacer" is quite common during the affine sorption. Nature "spacers," very diverse [Day, Hashim, 1984; Lowe, 1977; Martinez-Ceron et al., 2012]. We used a "spacer" composed of two amino acid residues (Asp-Asp). Introduction dipeptide took place in phosphate buffer (pH 7,8 - 8,0).

The test peptide was dissolved in double distilled water and added to the affinity resin under strict pH control, taking 5 mg peptide per 1 ml of adsorbent. "Crosslinking" was carried out at a temperature of 4 ° C for 12 hours. At the end of "crosslinking" of the process is not carefully washed by repeated washing the bound product with acetate buffer at pH 5.0 and bicarbonate buffer pH 9.0.

For preparations of the cerebral cortex of rats taking animals - Wistar rats, male, weighing about 300 g. Animals were decapitated, cold isolated cerebral cortex. The resulting samples were placed in liquid nitrogen, ground to a condition wherein the fine powder. The sample was then transferred to a 10 mM TRIS buffer pH 7.6 with 0.3 M sucrose (Helicon), 1mM CaCl₂ (Sigma-Aldrich) and 10 mM MgCl₂ (Sigma- Aldrich). Next, the resulting homogenate is centrifuged in the cold at 15,000 rev / min for 20 minutes.

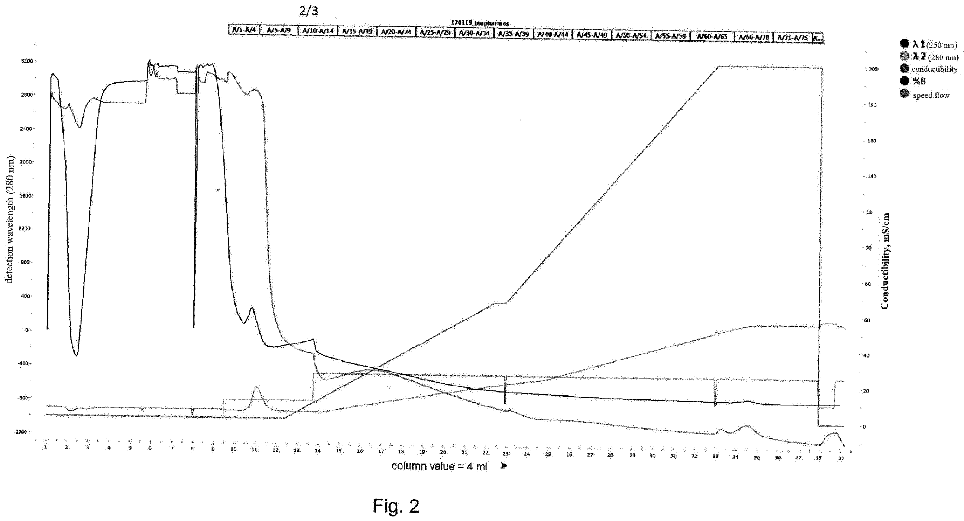

Affinity sorption was carried out for 24 hours at 4°C on a shaker with the supernatant obtained after centrifugation preparations cortex. Further, the sorbent was transferred to a column (Bio-Rad) and washed by non-specifically bound protein. The washing was carried out with Tris buffer at pH 7.6 without sucrose. washing out protein was monitored by GC BioRad NGC Discovery. Elution looked at λ = 280 nm. The protein is then washed with TRIS-buffer, pH 7.6, containing NaCl. The amount of salt according to the given gradient from 0.05 M to 0.5 M. The detection was performed at 280 nm, flow rate 0.1 ml/min. Figure 2 show the presence of two peaks 32 to 34 minutes, that will indicate the presence of the protein fraction, specifically bound to the peptide molecule.

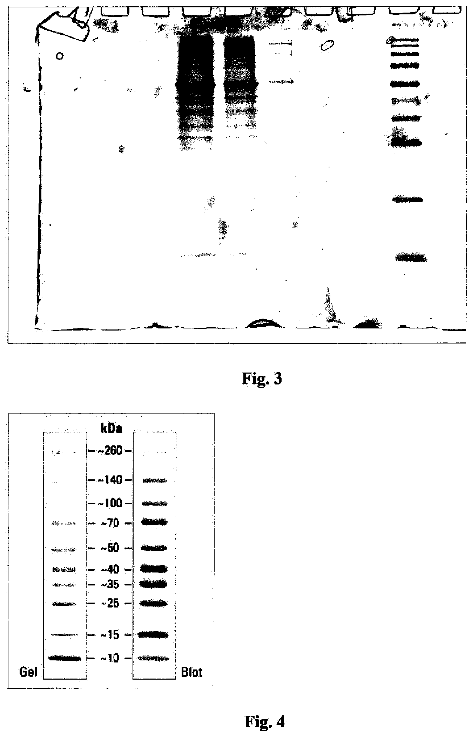

The protein fraction, specifically bound to the peptide molecule, subsequently subjected to fractionation by electrophoresis and direct detection of the individual protein molecules. Electrophoresis was performed in PAAG-gel. Concentrated fraction used in amounts of 1, 5 and 10 microliters. Spectra applied marker (Spectra ™ Multicolor Broad Range Protein Ladder). Figure 3 clearly shows two major protein compound is applied to the right marker left- fraction studied at three concentrations. To identify the three strips have been cut, the corresponding masses of marker 260, 50 and 10 kDa. To determine the mass of the test pattern used producer guide (Fig.4).

Identification phoresis spots produced by means of mass spectrometry. Chromato-mass spectrometry analysis was performed on tryptic peptides isolated from the cut gel pieces with the protein after enzyme treatment with trypsin. Then, chromato-mass spectrometry of this peptides solution and identification of peptide sequences by database were carried out. After identifying the results were validated using auto validation procedure. Peptide sequences of trypsin were the quality control. To verify the identification of spots, a search was performed on the mammalian databases. The top spot corresponding to the aisles weight from 140 kDa to 260 kDa was identified as clathrin heavy chain of 13 peptide sequences. As was previously established and proven analgesic effect of the test peptides and CME inhibition by blocking clathrin may stop or limit the transmission of pain signals, it was concluded that the studied peptides are specific inhibitors of clathrin heavy chain.

It should be noted that the elution (separation) of clathrin heavy chain peptide occurred at high values of the gradient, indicating a substantial affinity to the peptide clathrin heavy chain.

Consequently, the present invention can also be used as a method of allocating clathrin heavy chain. In this allocation method is much simpler than the conventional [Riddelle-Spencer, O'Halloran, 1997]. Therefore, the proposed method can be used for preparative separation of clathrin heavy chain of molecular biology and nanotechnology research [Santos dos et al., 2011].

Thus, the technical result of the invention is the simplicity and low cost of synthesis of the proposed peptides are specific inhibitors of clathrin and their lack of unwanted immunological activity and other side effects that allows to consider them as the basis for creating safe drugs which are specific inhibitors of clathrin and derived simple synthesis.

### Reference

1. McMahon NT, Boucrot E. Molecular mechanism and physiological functions of clathrin-mediated endocytosis //Nat Rev Mol Cell Biol. 2011. V. 12. Nº8. pp. 517-533.
2. Hood FE, Royle SJ Functional equivalence of the clathrin heavy chains CHC17 and CHC22 in endocytosis and mitosis // J. Cell Sci. 2009. V. 122. Nº13. pp. 2185-2190.
3. McMahon HT, Boucrot E. Molecular mechanism and physiological functions of clathrin-mediated endocytosis //Nat Rev Mol Cell Biol. 2011. V. 12. Nº8. pp. 517-533.
4. Haar E. ter, Harrison SC, Kirchhausen T. Peptide-in-groove interactions link target proteins to the beta-propeller of clathrin. // Proc. Natl. Acad. Sci. USA 2000. V. 97. Nº3. pp. 1096-1100.
5. Most P. et al. Extracellular S100A1 Protein Inhibits Apoptosis in Ventricular Cardiomyocytes via Activation of the Extracellular Signal-regulated Protein Kinase 1/2 (ERK1 / 2) // J. Biol. Chem. 2003. V. 278. Nº48. pp. 48404-48412.
6. Pellett S. et al. Botulinum neurotoxins can enter cultured neurons independent of synaptic vesicle recycling // PLoS One. 2015. V. 10. Nº7. pp. 1-17.
7. No. WO2013010218 A9. Inhibition of clathrin / Volker H., Robinson P., Mccluskey A. Publ. 24.01.2013.
8. Robertson MJ et al. Synthesis of the Pitstop family of clathrin inhibitors. //Nat. Protoc. 2014. V9. pp. 1592.
9. Willox AK, Sahraoui YME, Royle SJ Non-specificity of Pitstop 2 in clathrin-mediated endocytosis. // Biol. Open. 2014. V. 3. Nº5. pp. 326-31.
10. Patent JP201 1093852A. Clathrin-bonding peptide derivative / Kitahara H. et al. Publ. 12.05.2011.
11. PCT WO / 2013/141750 "Synthetic peptides with a non-narcotic type of analgesic effect", 2013
12. RF Patent 2508295 synthetic peptides with non-narcotic type analgesic action / Vlasov GP, Kotin AM / Bull. - 2013 - Nº27.
13. Aridor M., Hannan L. a. Traffic jams II: an update of diseases of intracellular transport. // Traffic. 2002. V. 3. Nº11. pp. 781-790.
14. Aridor M., Hannan LA Traffic Jam: A Compendium of Human Diseases that Affect Intracellular Transport Processes // Traffic. 2000. V. 1. Nº11. pp. 836-851.
15. Harold D. et al. Genome- Wide Association Study Identifies Variants at CLU and PICALM Associated with Alzheimer's Disease, and Shows Evidence for Additional Susceptibility Genes // Nat. Genet. 2009. V. 41. Nº10. pp. 1088-1093.
16. Edvardson S. et al. A deleterious mutation in DNAJC6 encoding the neuronal- specific clathrin-uncoating Co-chaperone auxilin, is associated with juvenile parkinsonism // PLoS One. 2012. V. 7. Nº5. pp. 4-8.
17. Casillas-Espinosa PM, Powell KL, O'Brien TJ Regulators of synaptic transmission: roles in the pathogenesis and treatment of epilepsy. // Epilepsia. 2012. V. 53 Suppl 9. pp. 41-58.
18. Nowack A. et al. Levetiracetam reverses synaptic deficits produced by overexpression of sV2A //PLoS One. 2011. V. 6. Nº12. pp. 1-8.
19. Richards BL, Whittle SL, Buchbinder R. Neuromodulators for pain management in rheumatoid arthritis. // Cochrane database Syst. Rev. V. 1. Nº1. pp. CD008921.
20. Nahorski MS et al. A novel disorder reveals clathrin heavy chain-22 is essential for human pain and touch development // Brain. 2015. 138. V. 138. Nº8. pp. 2147-2160.
21. Escobar GA et al. Clathrin heavy chain is required for TNF-induced inflammatory signaling // Surgery. 2006. V. 140. Nº2. pp. 268-272.
22. Kim ML, Sorg I., Arrieumerlou C. Endocytosis-independent function of clathrin heavy chain in the control of basal NF-κB activation // PLoS One. 2011. V. 6. Nº2.
23. Booth DG et al. A TACC3 / ch-TOG / clathrin complex stabilises kinetochore fibres by inter-microtubule bridging. // EMBO J. 2011. V. 30. Nº5. pp. 906-19.
24. Smith SM. et al. Inhibition of clathrin by pitstop 2 activates the spindle assembly checkpoint and induces cell death in dividing HeLa cancer cells. // Mol. Cancer. 2013. V.12. pp. 4.
25. Anantpadma M. et al. Large-Scale Screening and Identification of Novel Ebola Virus and Marburg Virus Entry Inhibitors // 2016. V. 60. Nº8. pp. 4471-4481.
26. Aleksandrowicz P. et al. Ebola virus enters host cells by macropinocytosis and clathrin-mediated endocytosis // J. Infect. Dis. 2011. V. 204. Nº SUPPL. 3. pp. 957-967.
27. Piccini LE, Castilla V., Damonte EB Dengue-3 virus entry into vero cells: Role of clathrin-mediated endocytosis in the outcome of infection // PLoS One. 2015. V. 10. Nº10. pp. 1-17.
28. Day ED, Hashim GA Affinity purification of two populations of antibodies against format determinants of synthetic myelin basic protein peptide S82 from S82-AH- and S82-CH-Sepharose 4B columns. // Neurochem. Res. 1984. V. 9. Nº10. pp. 1453-65.
29. Lowe CR Immobilised lipoamide dehydrogenase. 2. Properties of the enzyme immobilised to agarose through spacer molecules of various lengths. // Eur. J. Biochem. 1977. V. 76. Nº2. pp. 401-9.
30. Martinez-Ceron MC et al. Recombinant protein purification using complementary peptides as affinity tags //N. Biotechnol. 2012. V. 29. Nº2. pp. 206-210.
31. The official website of the company «Thermo Fisher Scientific)) / [electronic resource]. - Access: URL: https://www.thermofisher.com/order/catalog/product/26634 (reference date: 03.17.2017).
32. Riddelle-Spencer KS, O'Halloran TJ Purification of clathrin heavy and light chain from Dictyostelium discoideum. // Protein Expr. Purif. 1997. V. 11. Nº3. pp. 250-6.
33. Santos T. dos et al. Effects of transport inhibitors on the cellular uptake of carboxylated polystyrene nanoparticles in different cell lines. // PLoS One. 2011. V. 6. Nº9. pp. e24438.

SEQUENCE LISTING

<110> KOTIN O.A.
<120> SPECIFIC PEPTIDE CLATHRIN INHIBITORS
<160> 144
<210> SEQ ID NO 1
<211> 7
<212> Peptide
<213> Artificial sequence
<223>
<400> SEQUENCE: 1

Í- L-Leu- L-His- L-Lys- L-Leu- L-Gln- L-Thr- OMe
Í- L-Leu- L-His- L-Lys- L-Leu- L-GIn- L-Thr- NH2
Í- L-Ala- L-His- L-Lys- L-Leu- L-Gln- L-Thr- OMe
Í- L-Ala- L-His- L-Lys- L-Leu- L-GIn- L-Thr- NH2
Í- D-Ala- L-His- L-Lys- L-Leu- L-Gln- L-Thr- OMe
Í- D-AIa- L-His- L-Lys- L-Lcu- L-GIn- L-Thr- NH2
Í- L-Leu- D-His- L-Lys- L-Leu- L-Gln- L-Thr- OMe
Í- L-Leu- D-His- L-Lys- L-Leu- L-Gln- L-Thr- NH2
Í- L-Ala- D-His- L-Lys- L-Leu- L-Gln- L-Thr- OMe
Í- L-Ala- D-His- L-Lys- L-Leu- L-Gln- L-Thr- NH2
Í- D-Ala- D-His- L-Lys- L-Leu- L-Gln- L-Thr- OMe
H- D-Ala- D-His- L-Lys- L-Leu- L-Gln- L-Thr- NH2
H- L-Leu- L-Ala- L-Lys- L-Leu- L-Gln- L-Thr- OMe
H- L-Leu- L-Ala- L-Lys- L-Leu- L-Gln- L-Thr- NH2
H- L-Ala- L-Ala- L-Lys- L-Leu- L-Gln- L-Thr- OMe
H- L-Ala- L-Ala- L-Lys- L-Leu- L-Gln- L-Thr- NH2
H- D-Ala- L-Ala- L-Lys- L-Leu- L-Gln- L-Thr- OMe
H- D-Ala- L-Ala- L-Lys- L-Leu- L-Gln- L-Thr- NH2
H- L-Leu- D-Ala- L-Lys- L-Leu- L-Gln- L-Thr- OMe
H- L-Leu- D-Ala- L-Lys- L-Leu- L-Gln- L-Thr- NH2
H- L-Ala- D-Ala- L-Lys- L-Leu- L-Gln- L-Thr- OMe
H- L-Ala- D-Ala- L-Lys- L-Leu- L-Gln- L-Thr- NH2
H- D-Ala- D-Ala- L-Lys- L-Leu- L-Gln- L-Thr- OMe
H- D-Ala- D-Ala- L-Lys- L-Leu- L-Gln- L-Thr- NH2
H- L-Leu- L-His- L-Lys- L-Leu- L-Ala- L-Thr- OMe
H- L-Leu- L-His- L-Lys- L-Leu- L-Ala- L-Thr- NH2
H- L-Ala- L-His- L-Lys- L-Leu- L-Ala- L-Thr- OMe
H- L-Ala- L-His- L-Lys- L-Leu- L-Ala- L-Thr- NH2
H- D-Ala- L-His- L-Lys- L-Leu- L-Ala- L-Thr- OMe
H- D-Ala- L-His- L-Lys- L-Leu- L-Ala- L-Thr- NH2
H- L-Leu- D-His- L-Lys- L-Leu- L-Ala- L-Thr- OMe
H- L-Leu- D-His- L-Lys- L-Leu- L-Ala- L-Thr- NH2
H- L-Ala- D-His- L-Lys- L-Leu- L-Ala- L-Thr- OMe
H- L-Ala- D-His- L-Lys- L-Leu- L-Ala- L-Thr- NH2
H- D-Ala- D-His- L-Lys- L-Leu- L-Ala- L-Thr- OMe
H- D-Ala- D-His- L-Lys- L-Leu- L-Ala- L-Thr- NH2
H- L-Leu- L-Ala- L-Lys- L-Leu- L-Ala- L-Thr- OMe
H- L-Leu- L-Ala- L-Lys- L-Leu- L-Ala- L-Thr- NH2
H- L-Ala- L-Ala- L-Lys- L-Leu- L-Ala- L-Thr- OMe
H- L-Ala- L-Ala- L-Lys- L-Leu- L-Ala- L-Thr- NH2
H- D-Ala- L-Ala- L-Lys- L-Leu- L-Ala- L-Thr- OMe
H- D-Ala- L-Ala- L-Lys- L-Leu- L-Ala- L-Thr- NH2
H- L-Leu- D-AIa- L-Lys- L-Leu- L-Ala- L-Thr- OMe
H- L-Leu- D-AIa- L-Lys- L-Leu- L-Ala- L-Thr- NH2
H- L-Ala- D-Ala- L-Lys- L-Leu- L-Ala- L-Thr- OMe
H- L-Ala- D-Ala- L-Lys- L-Leu- L-Ala- L-Thr- NH2
H- D-Ala- D-Ala- L-Lys- L-Leu- L-Ala- L-Thr- OMe
H- D-Ala- D-Ala- L-Lys- L-Leu- L-Ala- L-Thr- NH2
H- L-Leu- L-His- L-Lys- L-Leu- D-Ala- L-Thr- OMe
H- L-Leu- L-His- L-Lys- L-Leu- D-Ala- L-Thr- NH2
H- L-Ala- L-His- L-Lys- L-Leu- D-Ala- L-Thr- OMe

H- L-Ala- L-His- L-Lys- L-Leu- D-Ala- L-Thr- NH2
H- D-Ala- L-His- L-Lys- L-Leu- D-Ala- L-Thr- OMe
H- D-Ala- L-His- L-Lys- L-Leu- D-Ala- L-Thr- NH2
H- L-Leu- D-His- L-Lys- L-Leu- D-Ala- L-Thr- OMe
H- L-Leu- D-His- L-Lys- L-Leu- D-Ala- L-Thr- NH2
H- L-Ala- D-His- L-Lys- L-Leu- D-Ala- L-Thr- OMe
H- L-Ala- D-His- L-Lys- L-Leu- D-Ala- L-Thr- NH2
H- D-Ala- D-His- L-Lys- L-Leu- D-Ala- L-Thr- OMe
H- D-Ala- D-His- L-Lys- L-Leu- D-Ala- L-Thr- NH2
H- L-Leu- L-Ala- L-Lys- L-Leu- D-Ala- L-Thr- OMe
H- L-Leu- L-Ala- L-Lys- L-Leu- D-Ala- L-Thr- NH2
H- L-Ala- L-Ala- L-Lys- L-Leu- D-Ala- L-Thr- OMe
H- L-Ala- L-Ala- L-Lys- L-Leu- D-Ala- L-Thr- NH2
H- D-Ala- L-Ala- L-Lys- L-Leu- D-Ala- L-Thr- OMe
H- D-Ala- L-Ala- L-Lys- L-Leu- D-Ala- L-Thr- NH2
H- L-Leu- D-Ala- L-Lys- L-Leu- D-Ala- L-Thr- OMe
H- L-Leu- D-Ala- L-Lys- L-Leu- D-Ala- L-Thr- NH2
H- L-Ala- D-Ala- L-Lys- L-Leu- D-Ala- L-Thr- OMe
H- L-AIa- D-Ala- L-Lys- L-Leu- D-Ala- L-Thr- NH2
H- D-Ala- D-Ala- L-Lys- L-Leu- D-Ala- L-Thr- OMe
H- D-Ala- D-Ala- L-Lys- L-Leu- D-Ala- L-Thr- NH2
H- L-Tyr- L-Leu- L-His- L-Lys- L-Leu- L-Gln- L-Thr- OMe
H- L-Tyr- L-Leu- L-His- L-Lys- L-Leu- L-Gln- L-Thr- NH2
H- L-Tyr- L-Ala- L-His- L-Lys- L-Leu- L-Gln- L-Thr- OMe
H- L-Tyr- L-Ala- L-His - L-Lys- L-Leu- L-Gln- L-Thr- NH2
H- L-Tyr- D-Ala- L- His - L-Lys- L-Leu- L-Gln- L-Thr- OMe
H- L-Tyr- D-Ala- L- His - L-Lys- L-Leu- L-Gln- L-Thr- NH2
H- L-Tyr- L-Leu- D-His - L-Lys- L-Leu- L-Gln- L-Thr- OMe
H- L-Tyr- L-Leu- D-His - L-Lys- L-Leu- L-Gln- L-Thr- NH2
H- L-Tyr- L-Ala- D-His - L-Lys- L-Leu- L-Gln- L-Thr- OMe
H- L-Tyr- L-Ala- D-His - L-Lys- L-Leu- L-Gln- L-Thr- NH2
H- L-Tyr- D-Ala- D-His - L-Lys- L-Leu- L-Gln- L-Thr- OMe
H- L-Tyr- D-Ala- D-His- L-Lys- L-Leu- L-Gln- L-Thr- NH2
H- L-Tyr- L-Leu- L-Ala- L-Lys- L-Leu- L-Gln- L-Thr- OMe
H- L-Tyr- L-Leu- L-Ala- L-Lys- L-Leu- L-Gln- L-Thr- NH2
H- L-Tyr- L-Ala- L-Ala- L-Lys- L-Leu- L-Gln- L-Thr- OMe
H- L-Tyr- L-Ala- L-Ala- L-Lys- L-Leu- L-Gln- L-Thr- NH2
H- L-Tyr- D-Ala- L-Ala- L-Lys- L-Leu- L-Gln- L-Thr- OMe
H- L-Tyr- D-Ala- L-Ala- L-Lys- L-Leu- L-Gln- L-Thr- NH2
H- L-Tyr- L-Leu- D-Ala- L-Lys- L-Leu- L-Gln- L-Thr- OMe
H- L-Tyr- L-Leu- D-Ala- L-Lys- L-Leu- L-Gln- L-Thr- NH2
H- L-Tyr- L-Ala- D-Ala- L-Lys- L-Leu- L-Gln- L-Thr- OMe
H- L-Tyr- L-Ala- D-Ala- L-Lys- L-Leu- L-Gln- L-Thr- NH2
H- L-Tyr- D-Ala- D-Ala- L-Lys- L-Leu- L-Gln- L-Thr- OMe
H- L-Tyr- D-Ala- D-Ala- L-Lys- L-Leu- L-Gln- L-Thr- NH2
H- L-Tyr- L-Leu- L-His- L-Lys- L-Leu- L-Ala- L-Thr- OMe
H- L-Tyr- L-Leu- L-His- L-Lys- L-Leu- L-Ala- L-Thr- NH2
H- L-Tyr- L-Ala- L-His- L-Lys- L-Leu- L-AIa- L-Thr- OMe
H- L-Tyr- L-Ala- L-His- L-Lys- L-Leu- L-Ala- L-Thr- NH2
H- L-Tyr- D-Ala- L-His- L-Lys- L-Leu- L-Ala- L-Thr- OMe
H- L-Tyr- D-Ala- L-His- L-Lys- L-Leu- L-Ala- L-Thr- NH2
H- L-Tyr- L-Leu- D-His- L-Lys- L-Leu- L-Ala- L-Thr- OMe
H- L-Tyr- L-Leu- D-His- L-Lys- L-Leu- L-Ala- L-Thr- NH2
H- L-Tyr- L-Ala- D-His - L-Lys- L-Leu- L-Ala- L-Thr- OMe
H- L-Tyr- L-Ala- D-His - L-Lys- L-Leu- L-Ala- L-Thr- NH2
H- L-Tyr- D-Ala- D-His - L-Lys- L-Leu- L-Ala- L-Thr- OMe
H- L-Tyr- D-Ala- D-His- L-Lys- L-Leu- L-Ala- L-Thr- NH2
H- L-Tyr- L-Leu- L-Ala- L-Lys- L-Leu- L-Ala- L-Thr- OMe
H- L-Tyr- L-Leu- L-Ala- L-Lys- L-Leu- L-Ala- L-Thr- NH2
H- L-Tyr- L-Ala- L-Ala- L-Lys- L-Leu- L-Ala- L-Thr- OMe
H- L-Tyr- L-Ala- L-Ala- L-Lys- L-Leu- L-Ala- L-Thr- NH2
H- L-Tyr- D-Ala- L-Ala- L-Lys- L-Leu- L-Ala- L-Thr- OMe
H- L-Tyr- D-Ala- L-Ala- L-Lys- L-Leu- L-Ala- L-Thr- NH2

H- L-Tyr- L-Leu- D-Ala- L-Lys- L-Leu- L-Ala- L-Thr- OMe
H- L-Tyr- L-Leu- D-Ala- L-Lys- L-Leu- L-Ala- L-Thr- NH2
H- L-Tyr- L-Ala- D-Ala- L-Lys- L-Leu- L-Ala- L-Thr- OMe
H- L-Tyr- L-Ala- D-Ala- L-Lys- L-Leu- L-Ala- L-Thr- NH2
H- L-Tyr- D-Ala- D-Ala- L-Lys- L-Leu- L-Ala- L-Thr- OMe
H- L-Tyr- D-Ala- D-Ala- L-Lys- L-Leu- L-Ala- L-Thr- NH2
H- L-Tyr- L-Leu- L-His- L-Lys- L-Leu- D-Ala- L-Thr- OMe
H- L-Tyr- L-Leu- L-His- L-Lys- L-Leu- D-Ala- L-Thr- NH2
H- L-Tyr- L-Ala- L-His- L-Lys- L-Leu- D-Ala- L-Thr- OMe
H- L-Tyr- L-Ala- L-His- L-Lys- L-Leu- D-Ala- L-Thr- NH2
H- L-Tyr- D-Ala- L-His- L-Lys- L-Leu- D-Ala- L-Thr- OMe
H- L-Tyr- D-Ala- L-His- L-Lys- L-Leu- D-Ala- L-Thr- NH2
H- L-Tyr- L-Leu- D-His- L-Lys- L-Leu- D-Ala- L-Thr- OMe
H- L-Tyr- L-Leu- D-His- L-Lys- L-Leu- D-Ala- L-Thr- NH2
H- L-Tyr- L-Ala- D-His- L-Lys- L-Leu- D-Ala- L-Thr- OMe
H- L-Tyr- L-Ala- D-His- L-Lys- L-Leu- D-Ala- L-Thr- NH2
H- L-Tyr- D-Ala- D-His- L-Lys- L-Leu- D-Ala- L-Thr- OMe
H- L-Tyr- D-Ala- D-His- L-Lys- L-Leu- D-Ala- L-Thr- NH2
H- L-Tyr- L-Leu- L-Ala- L-Lys- L-Leu- D-Ala- L-Thr- OMe
H- L-Tyr- L-Leu- L-Ala- L-Lys- L-Leu- D-Ala- L-Thr- NH2
H- L-Tyr- L-Ala- L-Ala- L-Lys- L-Leu- D-Ala- L-Thr- OMe
H- L-Tyr- L-Ala- L-Ala- L-Lys- L-Leu- D-Ala- L-Thr- NH2
H- L-Tyr- D-Ala- L-Ala- L-Lys- L-Leu- D-Ala- L-Thr- OMe
H- L-Tyr- D-Ala- L-Ala- L-Lys- L-Leu- D-Ala- L-Thr- NH2
H- L-Tyr- L-Leu- D-Ala- L-Lys- L-Leu- D-Ala- L-Thr- OMe
H- L-Tyr- L-Leu- D-Ala- L-Lys- L-Leu- D-Ala- L-Thr- NH2
H- L-Tyr- L-Ala- D-Ala- L-Lys- L-Leu- D-Ala- L-Thr- OMe
H- L-Tyr- L-Ala- D-Ala- L-Lys- L-Leu- D-Ala- L-Thr- NH2
H- L-Tyr- D-Ala- D-Ala- L-Lys- L-Leu- D-Ala- L-Thr- OMe
H- L-Tyr- D-Ala- D-Ala- L-Lys- L-Leu- D-Ala- L-Thr- NH2

<110> KOTIN O.A.
<120> SPECIFIC PEPTIDE CLATHRIN INHIBITORS
<160> 144
<210> SEQ ID NO 2
<211> 7
<212> Peptide
<213> Artificial sequence
<223>
<400> SEQUENCE: 2

H- D-Thr- D-Gln- D-Leu- D-Lys- D-His- D-Leu- OMe
H- D-Thr- D-Gln- D-Leu- D-Lys- D-His- D-Leu- NH2
H- D-Thr- D-Gln- D-Leu- D-Lys- D-His- D-Ala- OMe
H- D-Thr- D-Gln- D-Leu- D-Lys- D-His- D-Ala- NH2
H- D-Thr- D-Gln- D-Leu- D-Lys- D-His- L-Ala- OMe
H- D-Thr- D-Gln- D-Leu- D-Lys- D-His- L-Ala- NH2
H- D-Thr- D-Gln- D-Leu- D-Lys- L-His- D-Leu- OMe
H- D-Thr- D-Gln- D-Leu- D-Lys- L-His- D-Leu- NH2
H- D-Thr- D-Gln- D-Leu- D-Lys- L-His- D-Ala- OMe
H- D-Thr- D-Gln- D-Leu- D-Lys- L-His- D-Ala- NH2
H- D-Thr- D-Gln- D-Leu- D-Lys- L-His- L-Ala- OMe
H- D-Thr- D-Gln- D-Leu- D-Lys- L-His- L-Ala- NH2
H- D-Thr- D-Gln- D-Leu- D-Lys- D-Ala- D-Leu- OMe
H- D-Thr- D-Gln- D-Leu- D-Lys- D-Ala- D-Leu- NH2
H- D-Thr- D-Gln- D-Leu- D-Lys- D-Ala- D-Ala- OMe
H- D-Thr- D-Gln- D-Leu- D-Lys- D-Ala- D-Ala- NH2
H- D-Thr- D-Gln- D-Leu- D-Lys- D-Ala- L-Ala- OMe
H- D-Thr- D-Gln- D-Leu- D-Lys- D-Ala- L-Ala- NH2
H- D-Thr- D-Gln- D-Leu- D-Lys- L-Ala- D-Leu- OMe
H- D-Thr- D-Gln- D-Leu- D-Lys- L-Ala- D-Leu- NH2
H- D-Thr- D-Gln- D-Leu- D-Lys- L-Ala- D-Ala- OMe
H- D-Thr- D-Gln- D-Leu- D-Lys- L-Ala- D-Ala- NH2

H- D-Thr- D-Gln- D-Leu- D-Lys- L-Ala- L-Ala- OMe
H- D-Thr- D-Gln- D-Leu- D-Lys- L-AIa- L-Ala- NH2
H- D-Thr- D-Ala- D-Leu- D-Lys- D-His- D-Leu- OMe
H- D-Thr- D-Ala- D-Leu- D-Lys- D-His- D-Leu- NH2
H- D-Thr- D-Ala- D-Leu- D-Lys- D-His- D-Ala- OMe
H- D-Thr- D-Ala- D-Leu- D-Lys- D-His- D-Ala- NH2
H- D-Thr- D-Ala- D-Leu- D-Lys- D-His- L-Ala- OMe
H- D-Thr- D-Ala- D-Leu- D-Lys- D-His- L-Ala- NH2
H- D-Thr- D-Ala- D-Leu- D-Lys- L-His- D-Leu- OMe
H- D-Thr- D-Ala- D-Leu- D-Lys- L-His- D-Leu- NH2
H- D-Thr- D-Ala- D-Leu- D-Lys- L-His- D-Ala- OMe
H- D-Thr- D-Ala- D-Leu- D-Lys- L-His- D-AIa- NH2
H- D-Thr- D-Ala- D-Leu- D-Lys- L-His- L-Ala- OMe
H- D-Thr- D-Ala- D-Leu- D-Lys- L-His- L-Ala- NH2
H- D-Thr- D-Ala- D-Leu- D-Lys- D-Ala- D-Leu- OMe
H- D-Thr- D-Ala- D-Leu- D-Lys- D-Ala- D-Leu- NH2
H- D-Thr- D-Ala- D-Leu- D-Lys- D-Ala- D-Ala- OMe
H- D-Thr- D-Ala- D-Leu- D-Lys- D-Ala- D-AIa- NH2
H- D-Thr- D-Ala- D-Leu- D-Lys- D-Ala- L-Ala- OMe
H- D-Thr- D-Ala- D-Leu- D-Lys- D-Ala- L-Ala- NH2
H- D-Thr- D-Ala- D-Leu- D-Lys- L-Ala- D-Leu- OMe
H- D-Thr- D-Ala- D-Leu- D-Lys- L-Ala- D-Leu- NH2
H- D-Thr- D-Ala- D-Leu- D-Lys- L-Ala- D-Ala- OMe
H- D-Thr- D-Ala- D-Leu- D-Lys- L-Ala- D-Ala- NH2
H- D-Thr- D-Ala- D-Leu- D-Lys- L-Ala- L-Ala- OMe
H- D-Thr- D-Ala- D-Leu- D-Lys- L-Ala- L-Ala- NH2
H- D-Thr- L-Ala- D-Leu- D-Lys- D-His- D-Leu- OMe
H- D-Thr- L-Ala- D-Leu- D-Lys- D-His- D-Leu- NH2
H- D-Thr- L-Ala- D-Leu- D-Lys- D-His- D-Ala- OMe
H- D-Thr- L-Ala- D-Leu- D-Lys- D-His- D-Ala- NH2
H- D-Thr- L-Ala- D-Leu- D-Lys- D-His- L-Ala- OMe
H- D-Thr- L-Ala- D-Leu- D-Lys- D-His- L-Ala- NH2
H- D-Thr- L-Ala- D-Leu- D-Lys- L-His- D-Leu- OMe
H- D-Thr- L-Ala- D-Leu- D-Lys- L-His- D-Leu- NH2
H- D-Thr- L-Ala- D-Leu- D-Lys- L-His- D-Ala- OMe
H- D-Thr- L-Ala- D-Leu- D-Lys- L-His- D-Ala- NH2
H- D-Thr- L-Ala- D-Leu- D-Lys- L-His- L-Ala- OMe
H- D-Thr- L-Ala- D-Leu- D-Lys- L-His- L-Ala- NH2
H- D-Thr- L-Ala- D-Leu- D-Lys- D-Ala- D-Leu- OMe
H- D-Thr- L-Ala- D-Leu- D-Lys- D-Ala- D-Leu- NH2
H- D-Thr- L-Ala- D-Leu- D-Lys- D-Ala- D-Ala- OMe
H- D-Thr- L-Ala- D-Leu- D-Lys- D-Ala- D-Ala- NH2
H- D-Thr- L-Ala- D-Leu- D-Lys- D-Ala- L-Ala- OMe
H- D-Thr- L-Ala- D-Leu- D-Lys- D-Ala- L-Ala- NH2
H- D-Thr- L-Ala- D-Leu- D-Lys- L-Ala- D-Leu- OMe
H- D-Thr- L-Ala- D-Leu- D-Lys- L-Ala- D-Leu- NH2
H- D-Thr- L-Ala- D-Leu- D-Lys- L-Ala- D-Ala- OMe
H- D-Thr- L-Ala- D-Leu- D-Lys- L-Ala- D-Ala- NH2
H- D-Thr- L-Ala- D-Leu- D-Lys- L-Ala- L-Ala- OMe
H- D-Thr- L-Ala- D-Leu- D-Lys- L-Ala- L-Ala- NH2
H- D-Thr- D-Gln- D-Leu- D-Lys- D-His- D-Leu- D-Tyr- OMe
H- D-Thr- D-Gln- D-Leu- D-Lys- D-His- D-Leu- D-Tyr- NH2
H- D-Thr- D-Gln- D-Leu- D-Lys- D-His- D-Ala- D-Tyr- OMe
H- D-Thr- D-Gln- D-Leu- D-Lys- D-His- D-Ala- D-Tyr- NH2
H- D-Thr- D-Gln- D-Leu- D-Lys- D-His- L-Ala- D-Tyr- OMe
H- D-Thr- D-Gln- D-Leu- D-Lys- D-His- L-Ala- D-Tyr- NH2
H- D-Thr- D-Gln- D-Leu- D-Lys- L-His- D-Leu- D-Tyr- OMe
H- D-Thr- D-Gln- D-Leu- D-Lys- L-His- D-Leu- D-Tyr- NH2
H- D-Thr- D-Gln- D-Leu- D-Lys- L-His- D-Ala- D-Tyr- OMe
H- D-Thr- D-Gln- D-Leu- D-Lys- L-His- D-Ala- D-Tyr- NH2
H- D-Thr- D-Gln- D-Leu- D-Lys- L-His- L-Ala- D-Tyr- OMe
H- D-Thr- D-Gln- D-Leu- D-Lys- L-His- L-Ala- D-Tyr- NH2
H- D-Thr- D-Gln- D-Leu- D-Lys- D-Ala- D-Leu- D-Tyr- OMe

H- D-Thr- D-Gln- D-Leu- D-Lys- D-Ala- D-Leu- D-Tyr- NH2
H- D-Thr- D-Gln- D-Leu- D-Lys- D-Ala- D-Ala- D-Tyr- OMe
H- D-Thr- D-Gln- D-Leu- D-Lys- D-Ala- D-Ala- D-Tyr- NH2
H- D-Thr- D-Gln- D-Leu- D-Lys- D-Ala- L-Ala- D-Tyr- OMe
H- D-Thr- D-Gln- D-Leu- D-Lys- D-Ala- L-Ala- D-Tyr- NH2
H- D-Thr- D-Gln- D-Leu- D-Lys- L-Ala- D-Leu- D-Tyr- OMe
H- D-Thr- D-Gln- D-Leu- D-Lys- L-Ala- D-Leu- D-Tyr- NH2
H- D-Thr- D-Gln- D-Leu- D-Lys- L-Ala- D-Ala- D-Tyr- OMe
H- D-Thr- D-Gln- D-Leu- D-Lys- L-Ala- D-Ala- D-Tyr- NH2
H- D-Thr- D-Gln- D-Leu- D-Lys- L-Ala- L-Ala- D-Tyr- OMe
H- D-Thr- D-Gln- D-Leu- D-Lys- L-Ala- L-Ala- D-Tyr- NH2
H- D-Thr- D-Ala- D-Leu- D-Lys- D-His- D-Leu- D-Tyr- OMe
H- D-Thr- D-Ala- D-Leu- D-Lys- D-His- D-Leu- D-Tyr- NH2
H- D-Thr- D-Ala- D-Leu- D-Lys- D-His- D-Ala- D-Tyr- OMe
H- D-Thr- D-Ala- D-Leu- D-Lys- D-His- D-Ala- D-Tyr- NH2
H- D-Thr- D-Ala- D-Leu- D-Lys- D-His- L-Ala- D-Tyr- OMe
H- D-Thr- D-Ala- D-Leu- D-Lys- D-His- L-Ala- D-Tyr- NH2
H- D-Thr- D-Ala- D-Leu- D-Lys- L-His- D-Leu- D-Tyr- OMe
H- D-Thr- D-Ala- D-Leu- D-Lys- L-His- D-Leu- D-Tyr- NH2
H- D-Thr- D-Ala- D-Leu- D-Lys- L-His- D-Ala- D-Tyr- OMe
H- D-Thr- D-Ala- D-Leu- D-Lys- L-His- D-Ala- D-Tyr- NH2
H- D-Thr- D-Ala- D-Leu- D-Lys- L-His- L-Ala- D-Tyr- OMe
H- D-Thr- D-Ala- D-Leu- D-Lys- L-His- L-Ala- D-Tyr- NH2
H- D-Thr- D-Ala- D-Leu- D-Lys- D-Ala- D-Leu- D-Tyr- OMe
H- D-Thr- D-Ala- D-Leu- D-Lys- D-Ala- D-Leu- D-Tyr- NH2
H- D-Thr- D-Ala- D-Leu- D-Lys- D-Ala- D-AIa- D-Tyr- OMe
H- D-Thr- D-Ala- D-Leu- D-Lys- D-Ala- D-Ala- D-Tyr- NH2
H- D-Thr- D-Ala- D-Leu- D-Lys- D-Ala- L-Ala- D-Tyr- OMe
H- D-Thr- D-Ala- D-Leu- D-Lys- D-Ala- L-Ala- D-Tyr- NH2
H- D-Thr- D-Ala- D-Leu- D-Lys- L-Ala- D-Leu- D-Tyr- OMe
H- D-Thr- D-Ala- D-Leu- D-Lys- L-Ala- D-Leu- D-Tyr- NH2
H- D-Thr- D-Ala- D-Leu- D-Lys- L-Ala- D-AIa- D-Tyr- OMe
H- D-Thr- D-Ala- D-Leu- D-Lys- L-Ala- D-Ala- D-Tyr- NH2
H- D-Thr- D-AIa- D-Leu- D-Lys- L-AIa- L-Ala- D-Tyr- OMe
H- D-Thr- D-AIa- D-Leu- D-Lys- L-Ala- L-Ala- D-Tyr- NH2
H- D-Thr- L-Ala- D-Leu- D-Lys- D-His- D-Leu- D-Tyr- OMe
H- D-Thr- L-Ala- D-Leu- D-Lys- D-His- D-Leu- D-Tyr- NH2
H- D-Thr- L-Ala- D-Leu- D-Lys- D-His- D-Ala- D-Tyr- OMe
H- D-Thr- L-Ala- D-Leu- D-Lys- D-His- D-Ala- D-Tyr- NH2
H- D-Thr- L-Ala- D-Leu- D-Lys- D-His- L-Ala- D-Tyr- OMe
H- D-Thr- L-AIa- D-Leu- D-Lys- D-His- L-Ala- D-Tyr- NH2
H- D-Thr- L-Ala- D-Leu- D-Lys- L-His- D-Leu- D-Tyr- OMe
H- D-Thr- L-Ala- D-Leu- D-Lys- L-His- D-Leu- D-Tyr- NH2
H- D-Thr- L-AIa- D-Leu- D-Lys- L-His- D-AIa- D-Tyr- OMe
H- D-Thr- L-Ala- D-Leu- D-Lys- L-His- D-Ala- D-Tyr- NH2
H- D-Thr- L-Ala- D-Leu- D-Lys- L-His- L-Ala- D-Tyr- OMe
H- D-Thr- L-Ala- D-Leu- D-Lys- L-His- L-AIa- D-Tyr- NH2
H- D-Thr- L-AIa- D-Leu- D-Lys- D-AIa- D-Leu- D-Tyr- OMe
H- D-Thr- L-Ala- D-Leu- D-Lys- D-Ala- D-Leu- D-Tyr- NH2
H- D-Thr- L-Ala- D-Leu- D-Lys- D-Ala- D-Ala- D-Tyr- OMe
H- D-Thr- L-Ala- D-Leu- D-Lys- D-AIa- D-Ala- D-Tyr- NH2
H- D-Thr- L-Ala- D-Leu- D-Lys- D-Ala- L-Ala- D-Tyr- OMe
H- D-Thr- L-Ala- D-Leu- D-Lys- D-Ala- L-Ala- D-Tyr- NH2
H- D-Thr- L-AIa- D-Leu- D-Lys- L-Ala- D-Leu- D-Tyr- OMe
H- D-Thr- L-Ala- D-Leu- D-Lys- L-AIa- D-Leu- D-Tyr- NH2
H- D-Thr- L-Ala- D-Leu- D-Lys- L-Ala- D-AIa- D-Tyr- OMe
H- D-Thr- L-Ala- D-Leu- D-Lys- L-Ala- D-Ala- D-Tyr- NH2
H- D-Thr- L-Ala- D-Leu- D-Lys- L-Ala- L-Ala- D-Tyr- OMe
H- D-Thr- L-Ala- D-Leu- D-Lys- L-Ala- L-Ala- D-Tyr- NH2

2

## Claims

1. The use of synthetic peptide of general formula (I) [SEQ ID NO: 1]
XDL - XDL1 - XDL2 - L-Lys - L-Leu - XDL3 - L-Thr - R2 (I),
wherein:
XDL - the absence of an amino acid or L-Tyr,
XDL1 - one of the amino acids: L-Leu, L-Ala or D-Ala,
XDL2 - one of the amino acids: L-His, D-His, L-Ala or D-Ala,
XDL3 - one of the amino acids: L-Gln, L-Ala or D-Ala;
R2 - OMe or NH2,
or peptide - retroinversion of formula (I), having a reverse amino acid sequence with the replacement of L-form amino acids to D-form and D-form amino acids to L-form, of the general formula (II) [SEQ ID NO: 2]
D-Thr - XDL4 - D-Leu - D-Lys - XDL5 - XDL6 - XDL7 - R2 (II)
wherein:
XDL4 - one of the amino acids: D-Gln, D-Ala or L-Ala;
XDL5 - one of the amino acids: D-His, L-His, D-Ala or L-Ala,
XDL6 - one of the amino acids: D-Leu, D-Ala or L-Ala,
XDL7 - the absence of an amino acid or D-Tyr,
R2 - OMe or NH2,
as a specific inhibitor of clathrin.

2. Method of preventing or treating a disease or condition in an animal comprising administering an effective amount of synthetic peptides of general formula (I) [SEQ ID NO: 1] or general formula (II) [SEQ ID NO: 2] or a prodrug thereof, or a physiologically acceptable salt or solvate of the aforementioned peptides or pharmaceutical composition containing the above peptides as specific inhibitors of clathrin.
